# EUROPÄISCHE PATENTSCHRIFT

(11) **EP 0 642 788 B1**
(45) Veröffentlichungstag und Bekanntmachung des Hinweises auf die Patenterteilung: **27.05.1998**
(21) Anmeldenummer: 94113192.2
(22) Anmeldetag: 24.08.1994
(51) Int. Cl.: A61K 31/135, A61K 47/38, A61K 9/26, A61K 9/20

(54) **Tramadolsalz enthaltende Arzneimittel mit verzögerter Wirkstofffreitsetzung**
Tramadol salt containing medicaments with sustained release of the active ingredient
Médicaments contenant de sel de tramadol avec une libération retardée d'ingrédient actif

(30) Priorität: 03.09.1993 DE 4329794
(43) Veröffentlichungstag der Anmeldung: 15.03.1995
(73) Patentinhaber: Grünenthal GmbH, D-52078 Aachen (DE)
(72) Erfinder: Johannes, Heinrich Antonius Bartholomäus, Dr., D-52080 Aachen (DE)

(56) Entgegenhaltungen:
- EP-A- 0 624 366
- DE-A- 4 315 525
- US-A- 4 389 393
- CHEMICAL ABSTRACTS, vol. 92, no. 8, 25. Februar 1980, Columbus, Ohio, US; abstract no. 64751, Seite 385 ; & JP-A-54 092 631 (KYOWA CO. LTD.) 23 Juli 1979
- DATABASE WPI Section Ch, Week 9238, Derwent Publications Ltd., London, GB; Class A96, AN 92-312372 & JP-A-04 217 925 (NIPPON SURFACTANT KOGYO KK) 7. August 1992

## Beschreibung

Die Erfindung betrifft Arzneimittel in Tablettenform zur oralen Applikation, aus denen ein feuchtigkeitsunempfindliches, physiologisch verträgliches Salz von Tramadol verzögert freigesetzt wird und die mindestens einen pharmazeutisch akzeptablen Matrixbildner enthalten.

Tramadolhydrochlorid - (1RS;2 RS)-2[(Dimethylamino)methyl] -1-(3-methoxyphenyl)cyclohexanol, Hydrochlorid - ist ein Analgetikum, das bei starken und mittelstarken Schmerzen wirksam ist. Alle zur Zeit im Markt befindlichen Arzneiformen setzen Tramadolhydrochlorid unverzögert frei, so daß diese Arzneimittel zur Erzielung einer guten therapeutischen Wirksamkeit bei anhaltenden Schmerzen 3- bis 4mal täglich vom Patienten eingenommen werden müssen. Für die Patienten würde es daher eine Erleichterung bedeuten, wenn die Verabreichungsfrequenz auf 1- bis 2mal täglich reduziert werden könnte.

Verschiedene prinzipielle Ausführungsformen von Retardzubereitungen sind dem Fachmann bekannt. So wird beispielsweise in dem bereits am 19. April 1960 angemeldeten Schutzrecht US 3,065,143 eine Retardtablette offenbart, die einen pharmazeutisch akzeptablen hydrophilen Gummi enthält, der schnell Wasser absorbiert und bei 37° C quillt und dessen Gewichtsanteil in der Tablette wenigstens ein Drittel beträgt. Wenn die Tablette in Kontakt mit dem wäßrigen Medium des Gastrointestinaltraktes gebracht wird, bildet sich eine weiche Gelbarriere auf der Tablettenoberfläche, die einen schnellen Zerfall der Tablette und eine schnelle Wirkstofffreigabe verhindert und einen langsamen Zerfall mit der Freigabe des Wirkstoffes über einen Zeitraum von mindestens 4 Stunden ermöglicht. Die Beispiele zeigen jedoch, daß die Wirkstofffreisetzung aus den Tabletten vom pH-Wert beeinflußt wird. Weiterhin wird beschrieben, daß zur Freisetzung eines Wirkstoffes die äußere Gelschicht durch die Bewegungen im Gastrointestinaltrakt abgeschert wird, wodurch der Wirkstoff aus dem Gel freigesetzt wird. Gleichzeitig bildet sich an der dann freien Tablettenoberfläche eine neue Gelschicht. Die Wirkstofffreisetzung wird daher auch durch mechanische Beanspruchung beeinflußt. Desweiteren wird beschrieben, daß die Freisetzungsgeschwindigkeit vom Gewichtsverhältnis Wirkstoff zu Gummi sowie vom Gehalt an hydrophilem Gummi in der Tablette abhängig ist.

In US 4,389,393 (Reexamination Certificate B 1 4,389,393) wird ein Trägermaterial für feuchtigkeitsempfindliche Wirkstoffe offenbart, das, zu einer festen Dosierungseinheit geformt und gepreßt, eine regelmäßige und verzögerte Wirkstofffreisetzung nach der Verabreichung zeigt. Das Trägermaterial besteht aus einer oder mehreren Hydroxypropylmethylcellulosen oder aus einer Mischung aus einer oder mehreren Hydroxypropylmethylcellulosen und bis zu 30 Gew.-% aus Methylcellulose, Natriumcarboxymethylcellulose und/oder einem anderen Celluloseether, wobei wenigstens eine der Hydroxymethylpropylcellulosen einen Methoxygehalt zwischen 16 und 24 Gew.-%, einen Hydroxypropoxygehalt zwischen 4 und 32 Gew.-% und ein numerisches mittleres Molekulargewicht von mindestens 50.000 aufweist. Das Trägermaterial ist zu 30 Gew.-% oder weniger in der Dosierungsform enthalten und bewirkt, daß wenigstens 4 Stunden für die Freisetzung von 94,4 % des feuchtigkeitsempfindlichen Wirkstoffes aus der Dosierungsform nach der Verabreichung erforderlich sind.

In Int. J. Pharm. Tech. & Prod. Mfr. 5, 1 (1984) werden hydrophile Matrices, insbesondere Hydroxypropylmethylcellulosen, für orale Dosierungsformen mit kontrollierter Wirkstofffreisetzung beschrieben. Auf den Seiten 4 bis 6 dieses Dokumentes wird ausgeführt, daß die Freisetzungsgeschwindigkeit eines Arzneistoffes sowohl von der Viskosität als auch vom Gewichtsanteil des eingesetzten Polymers abhängt. Desweiteren beeinflussen Größe und Form der Dosierungseinheit die Freisetzung, wohingegen praktisch keine Abhängigkeit vom Herstellungsprozeß durch Granulation oder Direkttablettierung besteht. Dagegen zeigen unterschiedliche Füllstoffe in der Rezeptur einen starken Einfluß auf die Wirkstofffreisetzung. Gemäß den Abbildungen 16 und 18 bewirken unlösliche Hilfsstoffe eine Beschleunigung der Freisetzung bis hin zur vollständigen Aufhebung des Effektes der kontrollierten Freisetzung, unabhängig davon, ob diese Stoffe, wie zum Beispiel mikrokristalline Cellulose, quellen können oder diese Stoffe wie zum Beispiel Calciumhydrogenphosphat, nicht quellbar sind.

Aus Int. J. Pharm. 40, 223 (1987) ist bekannt, daß die Freisetzungsgeschwindigkeit eines Wirkstoffes aus einer Retardtablette, deren Matrixbildner Hydroxypropylmethylcellulose ist, vom Gewichtsverhältnis Wirkstoff zu Hydroxymethylpropylcellulose abhängt. Je mehr dieses Verhältnis zu Gunsten des Wirkstoffes verschoben ist, desto höher ist die Freisetzungsgeschwindigkeit. In Zubereitungen, die mehr als 50 Gew.-% Füllstoff enthalten, wird die Freisetzungsgeschwindigkeit durch die Art der verwendeten Hilfsstoffe beeinflußt. Ein teilweiser Ersatz von Hydroxypropylmethylcellulose durch einen Füllstoff und damit verbunden eine Verringerung des Gehaltes an Hydroxypropylmethylcellulose in der Zubereitung führt zu einer Erhöhung der Freisetzungsgeschwindigkeit.

Die in J. Pharm. Sci. 57, 1292 (1968) beschriebenen Matrix-Retardtabletten führen bei einer Erhöhung der löslichen Anteile in der hydrophilen Matrix zu einer Erhöhung der Freisetzungsgeschwindigkeit.

Die der Erfindung zugrundeliegende Aufgabe bestand darin, Arzneimittel in Tablettenform zur oralen Applikation bereitzustellen, aus denen ein feuchtigkeitsunempfindliches, physiologisch verträgliches Salz von Tramadol, unabhängig vom pH-Wert der Freisetzungsumgebung und unabhängig von der Art und Menge der Füllstoffe verzögert freigesetzt wird. Ferner soll bei einer vorgegebenen Masse und Form der Tablette das Freisetzungsprofil unabhängig vom Wirkstoffgehalt und der Menge des Matrixbildners sein. Unter "Freisetzungsprofil" wird der freigesetzte Anteil des Wirkstoffes in Gewichtsprozent des gesamten Wirkstoffgehaltes aufgetragen gegen die Untersuchungszeit verstanden.

Es wurde gefunden, daß die an eine Tramadolsalz enthaltende Retardform gestellten hohen Anforderungen von einem ein feuchtigkeitsunempfindliches Tramadolsalz enthaltenden Arzneimittel in Tablettenform erfüllt wird, das einen ausgewählten pharmazeutisch akzeptablen Matrixbildner enthält.

Gegenstand der Erfindung sind dementsprechend Arzneimittel in Tablettenform mit verzögerter Wirkstofffreisetzung, enthaltend als Wirkstoff mindestens ein feuchtigkeitsunempfindliches, physiologisch verträgliches Salz von Tramadol und als pharmazeutisch akzeptablen Matrixbildner mindestens einen Celluloseether und/oder Celluloseester, der in einer 2 gew.-%igen wäßrigen Lösung bei 20° C eine Viskosität zwischen 3.000 und 150.000 mPas aufweist.

Vorzugsweise werden als pharmazeutisch akzeptable Matrixbildner Celluloseether und/oder Celluloseester eingesetzt, die in einer 2 gew.-%igen wäßrigen Lösung bei 20° C eine Viskosität zwischen 10.000 und 150.000 mPas besitzen. Besonders geeignete pharmazeutisch akzeptable Matrixbildner sind ausgewählt aus der Gruppe der Methylhydroxypropylcellulosen, Hydroxyethylcellulosen, Hydroxypropylcellulosen, Methylcellulosen, Ethylcellulosen und Carboxymethylcellulosen und insbesondere ausgewählt aus der Gruppe der Methylhydroxypropylcellulosen, Hydroxyethylcellulosen und Hydroxypropylcellulosen.

In den erfindungsgemäßen Arzneimitteln liegt der verzögert freizusetzende Wirkstoffgehalt vorzugsweise zwischen 10 und 85 Gew.-% und der Gehalt an pharmazeutisch akzeptablem Matrixbildner zwischen 10 und 40 Gew.-%. Besonders bevorzugt werden Arzneimittel mit einem verzögert freizusetzenden Wirkstoffgehalt zwischen 25 und 70 Gew.-% und einem Gehalt an pharmazeutisch akzeptablem Matrixbildner zwischen 10 und 40 Gew.-%.

In den erfindungsgemäßen Arzneimitteln können als weitere Bestandteile pharmazeutisch gebräuchliche Hilfsstoffe wie Füllstoffe, beispielsweise Lactose, mikrokristalline Cellulose oder Calciumhydrogenphosphat, sowie Gleit-, Schmier- und Fließregulierungsmittel, beispielsweise hochdisperses Siliciumdioxid, Talkum, Magnesiumstearat und/oder Stearinsäure, enthalten sein, deren Gesamtgehalt in der Tablette zwischen 0 und 80 Gew.-%, vorzugsweise zwischen 5 und 65 Gew.-% liegt.

Vielfach ist die Freisetzungsgeschwindigkeit eines Wirkstoffes in einem Arzneimittel vom pH-Wert abhängig. Dieser kann während der Gastrointestinalpassage des Arzneimittels in einem pH-Wert-Bereich von unter 1 bis etwa 8 schwanken. Diese Schwankungen können von einer einnehmenden Person zur anderen verschieden sein. Auch kann bei ein und derselben Person von einer Einnahme zur anderen ein unterschiedliches pH-Wert-Zeit-Profil während der Gastrointestinalpassage gegeben sein. Ist die Freisetzungsgeschwindigkeit des Wirkstoffes aus dem Arzneimittel vom pH-Wert abhängig, so kann dies zu unterschiedlichen Freisetzungsgeschwindigkeiten in-vivo führen. Die Freisetzungsprofile eines Tramadolsalzes aus einem erfindungsgemäßen Arzneimittel sind jedoch überraschenderweise unabhängig vom pH-Wert wie er physiologisch während der Gastrointestinalpassage auftreten kann. Die Freisetzungsprofile bei einem Umgebungs-pH-Wert von 1,2, 4,0 und 6,8 sind sowohl untereinander identisch als auch im Vergleich zur Freisetzung während eines pH-Wert-Zeit-Profils von pH 1,2 über pH 2,3 und pH 6,8 bis zu pH 7,2.

Im Gegensatz zu dem vorgenannten Stand der Technik ist die Freisetzungsgeschwindigkeit eines Tramadolsalzes aus einem erfindungsgemäßen Arzneimittel sowohl unabhängig von der in einer 2 gew.-%igen Lösung zwischen 3.000 und 150.000 mPas liegenden Viskosität des Matrixbildners als auch unabhängig vom Gehalt des Matrixbildners sowie des Füllmittels im Arzneimittel.

Desweiteren ist es für das Freisetzungsprofil einer erfindungsgemäßen Tramadolsalz enthaltenden Retardtablette unerheblich, ob bei ansonsten unveränderten Abmessungen und unveränderter Zusammensetzung, bezogen auf den Wirkstoff, den Matrixbildner und die fakultativen Bestandteile, als Füllstoff ein wasserlöslicher Füllstoff, beispielsweise Lactose, ein unlöslicher, in wäßrigem Medium nicht quellender Füllstoff, beispielsweise Calciumhydrogenphosphat, oder ein unlöslicher, in wäßrigem Medium quellender Füllstoff, beispielsweise mikrokristalline Cellulose, eingesetzt wird. Alle derartigen Arzneimittel haben deckungsgleiche Freisetzungsprofile.

Da insbesondere Tramadolhydrochlorid in wäßrigem Medium gut löslich ist und aufgrund des Standes der Technik der Anteil löslicher Bestandteile in der Arzneimittelzusammensetzung einen Einfluß auf die Freisetzungsgeschwindigkeit hat, sollten Zubereitungen mit unterschiedlichem Gehalt an einem Tramadolsalz unterschiedliche Freisetzungsprofile haben. Ebenso sollte aufgrund des Standes der Technik eine Änderung des Verhältnisses Tramadolsalz zu Matrixbildner zu einer Veränderung des Freisetzungsprofils führen. Überraschenderweise zeigte sich aber, daß erfindungsgemäße Arzneimittel mit unterschiedlichem Wirkstoffgehalt, in denen die Gesamtmenge an feuchtigkeitsunempfindlichen, physiologisch verträglichem Tramadolsalz und löslichem oder unlöslichem Füllstoff konstant gehalten wurde, bei ansonsten unveränderten Abmessungen, unveränderten Gesamtgewicht und unveränderter Zusammensetzung der Tablette, bezogen auf den Matrixbildner und die fakultativen Hilfsstoffe identische Freisetzungsprofile aufweisen.

Die erfindungsgemäßen Arzneimittel können sowohl als einfache Tablette als auch als überzogene Tablette, beispielsweise als Filmtablette oder Drageé vorliegen. Für die überzogenen Tabletten können ein oder mehrere Überzugsschichten verwendet werden. Als Überzugsmaterial eignen sich bekannte Methylhydroxypropylcellulosen, die das Freisetzungsprofil der erfindungsgemäßen Arzneimittel nur geringfügig beeinflussen. Dem Fachmann bekannte Diffusionsüberzüge, beispielsweise auf Basis von quellbaren, aber wasserunlöslichen Poly(meth)acrylaten, führen zu noch stärker gebremsten Wirkstofffreisetzungen aus erfindungsgemäßen Arzneimitteln. Der wirkstoffhaltige, den Wirkstoff retardiert freisetzende Tablettenkern mit einem Wirkstoffgehalt vorzugsweise zwischen 10 und 85 Gew.-%, besonders bevorzugt zwischen 25 und 70 Gew.-%, kann mit zusätzlichen Wirkstoff, der nicht retardiert als Initialdosis freigesetzt wird, durch verschiedene, dem Fachmann bekannte Verfahren, beispielsweise Dragieren, Aufsprühen aus Lösungen oder Suspensionen oder durch Pulverauftragverfahren umhüllt sein. Weitere Ausführungsformen stellen Mehrschicht- und Manteltabletten dar, bei denen mindestens ein Tramadolsalz in einer oder mehreren Schichten der Mehrschichttablette mit einem Wirkstoffgehalt vorzugsweise zwischen 10 und 85 Gew.-%, besonders bevorzugt zwischen 25 und 70 Gew.-% bzw. im Kern der Manteltablette mit einem Wirkstoffgehalt vorzugsweise zwischen 10 und 85 Gew.-%, besonders bevorzugt zwischen 25 und 70 Gew.-% durch einen pharmazeutisch akzeptablen Matrixbildner retardiert freigesetzt wird und die Freisetzung eines Tramadolsalzes in einer oder mehreren Schichten der Mehrschichttablette bzw. der äußeren Mantelschicht der Manteltabletten unretardiert erfolgt. Mehrschicht- und Manteltabletten können ein oder mehrere wirkstofffreie Überzüge enthalten.

Die Herstellung erfindungsgemäßer Arzneimittel ist durch eine hohe Reproduzierbarkeit der Freisetzungseigenschaften der erhaltenen tramadolsalzhaltigen Zusammensetzungen gekennzeichnet. Während einer Lagerzeit von mindestens einem Jahr tritt keine Änderung des Freisetzungsprofils erfindungsgemäßer Arzneimittel ein.

Bei täglich einmaliger oder zweimaliger Einnahme einer erfindungsgemäßen Tablette durch den Patienten wird eine gute therapeutische Wirksamkeit bei anhaltend starken Schmerzen erzielt.

### Beispiele

### Beispiel 1

Matrixtabletten mit folgender Zusammensetzung pro Tablette

| | |
|---|---|
| Tramadolhydrochlorid | 100 mg |
| Methylhydroxypropylcellulose Type 2208, 100.000 mPas (Hersteller Dow Chemical Company, Midland/USA) | 85 mg |
| Calciumhydrogenphosphat | 62 mg |
| hochdisperses Siliciumdioxid | 5 mg |
| Magnesiumstearat | 3 mg |

wurden in einer Ansatzgröße von 200 g in folgender Weise hergestellt:

Alle Bestandteile wurden durch ein 0,63 mm Sieb gegeben, in einem Kubusmischer 10 Minuten gemischt und auf einer Korsch EK 0 Exzentertablettenpresse zu Tabletten mit einem Durchmesser von 9 mm, einem Wölbungsradius von 8,5 mm und einem mittleren Gewicht von 255 mg gepreßt.

In gleicher Weise wurden Matrixtabletten mit einem Gewicht von 255 mg pro Tablette und folgende Zusammensetzung pro Tablette hergestellt:

| | |
|---|---|
| Tramadolhydrochlorid | 150 mg |
| Methylhydroxypropylcelulose Type 2208, 100 000 mPas | 85 mg |
| Calciumhydrogenphosphat | 12 mg |
| hochdisperses Siliciumdioxid | 5 mg |
| Magnesiumstearat | 3 mg |

Die in-vitro-Freisetzung von Tramadolhydrochlorid aus den Arzneimittelzubereitungen wurde nach DAB 10 in einer Blattrührerapparatur bestimmt. Die Temperatur des Lösungsmediums betrug 37° C und die Umdrehungsgeschwindigkeit des Rührers 75 Umdrehungen/Minute. Zu Beginn der Untersuchung wurde jede Tablette in jeweils 600 ml künstlichen Magensaft mit einem pH-Wert von 1,2 gegeben. Nach 30 Minuten wurde durch Zugabe von Natronlauge der pH-Wert auf 2,3, nach weiteren 90 Minuten auf 6,5 und nach nochmals 60 weiteren Minuten auf 7,2 erhöht. Die zu den vorgenannten Zeitpunkten im Lösungsmedium befindliche freigesetzte Wirkstoffmenge wurde spektralphotometrisch bestimmt. Es wurden folgende Freisetzungswerte (Mittelwerte aus n = 3) ermittelt:

| Zeit in Minuten | Freigesetzter Anteil in Gew.-% enthaltend | |
|---|---|---|
| | 100 mg Tramadolhydrochlorid | 150 mg Tramadolhydrochlorid |
| 30 | 26 | 25 |
| 60 | 39 | 37 |
| 120 | 57 | 56 |
| 300 | 84 | 86 |
| 720 | 99 | 98 |

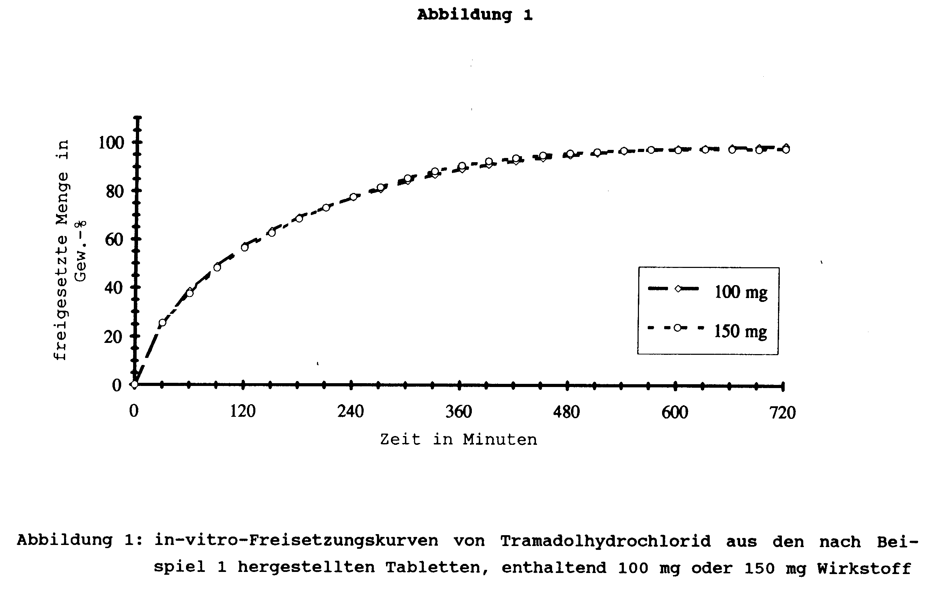

Die in-vitro Freisetzungskurven der Tabletten enthaltend 100 mg oder 150 mg Tramadolhydrochlorid sind in Abbildung 1 dargestellt.

### Beispiel 2

Matrixtabletten mit folgender Zusammensetzung pro Tablette:

| | |
|---|---|
| Tramadolhydrochlorid | 200 mg |
| Methylhydroxypropylcellulose Type 2208, 100.000 mPas (Hersteller: Shin Etsu, Tokio/Japan) | 105 mg |
| Calciumhydrogenphosphat | 36 mg |
| Hochdisperses Silicumdioxid | 5 mg |
| Magnesiumstearat | 4 mg |

wurden in einer Ansatzgröße von 525 g in folgender Weise hergestellt:

Tramadolhydrochlorid, Methylhydroxypropylcellulose, Calciumhydrogenphosphat sowie je 50 % der gesamten Menge an Siliciumdioxid und Magnesiumstearat wurden durch ein 0,5 mm Sieb gegeben und in einem Kubusmischer 10 Minuten gemischt. Die erhaltene Mischung wurde auf einer Korsch EK 0 Exzentertablettenpresse zu Preßlingen mit einem Durchmesser von 20 mm kompaktiert.

Nach Brechen der erhaltenen Preßlinge mittels eines 1 mm Siebes wurden die restlichen Mengen an Siliciumdioxid und Magnesiumstearat zugemischt. Anschließend wurde die erhaltene Mischung auf einer Korsch EK 0 Exzentertablettenpresse zu Tabletten mit einem Durchmesser von 10 mm, einem Wölbungsradius von 8 mm und einem mittleren Gewicht von 350 mg gepreßt.

Die in-vitro-Freisetzung des Wirkstoffes wurde gemäß Beispiel 1 untersucht. Folgende Freisetzungswerte (Mittelwerte aus n = 2) wurden erhalten:

| Zeit in Minuten | Freigesetzter Anteil in Gew.-% |
|---|---|
| 30 | 22 |
| 60 | 32 |
| 120 | 48 |
| 300 | 76 |
| 720 | 100 |

### Beispiel 3

Die nach Beispiel 2 hergestellten Tabletten wurden mittels Wursterverfahren mit einer Lacksuspension folgender Zusammensetzung überzogen:

| | |
|---|---|
| Eudragit RL30D (Hersteller Röhm, Darmstadt) | 18,2 Gew.-% |
| Talkum | 8,2 Gew.-% |
| Titandioxid | 6,5 Gew.-% |
| Polyethylenglykol 6000 (Hersteller: Hoechst AG, Frankfurt) | 1,6 Gew.-% |
| Triethylcitrat | 1,1 Gew.-% |
| demineralisiertes Wasser | 64,4 Gew.-% |

Das Durchschnittsgewicht der eingesetzten Tablettenkerne wurde durch den Lackauftrag um 20 mg erhöht. Die in-vitro-Freisetzung des Wirkstoffes aus den Filmtabletten wurde gemäß Beispiel 1 untersucht. Es wurden folgende Freisetzungswerte (Mittelwerte aus n = 2) erhalten:

| Zeit in Minuten | Freigesetzter Anteil in Gew.-% |
|---|---|
| 30 | 10 |
| 60 | 22 |
| 120 | 39 |
| 300 | 69 |
| 720 | 96 |

### Beispiel 4

Wie in Beispiel 2 beschrieben, wurden Tabletten mit einem mittleren Gewicht von 350 mg hergestellt, in denen jedoch Calciumhydrogenphosphat durch 36 mg mikrokristalline Cellulose PH 101 (Hersteller: FMC, Philadelphia/USA) und Methylhydroxypropylcellulose entweder durch 105 mg Methylhydroxypropylcellulose Type 2208 mit einer Viskosität von 15.000 mPas (Hersteller: Shin Etsu) oder durch 105 mg Methylhydroxypropylcellulose Type 2208 mit einer Viskosität von 50.000 mPas (Hersteller: Shin Etsu) ersetzt wurden. Die in-vitro-Freisetzungen des Wirkstoffes wurden gemäß Beispiel 1 untersucht. Es wurden folgende Freisetzungswerte (Mittelwerte aus n = 3) erhalten:

| Zeit in Minuten | Freigesetzter Anteil in Gew.-% der Tablette, enthaltend den Matrixbildner mit der Viskosität | |
|---|---|---|
| | 15 000 mPas | 50 000 mPas |
| 30 | 23 | 23 |
| 60 | 35 | 34 |
| 120 | 51 | 50 |
| 300 | 79 | 79 |
| 720 | 103 | 103 |

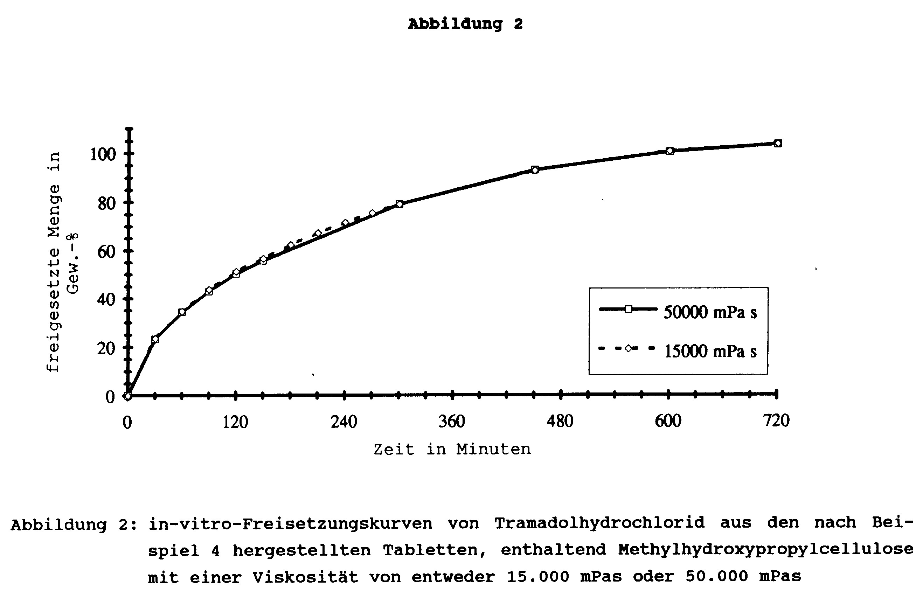

Die in-vitro-Freisetzungskurven der Tablettenzubereitungen, die Methylhydroxypropylcellulosen mit einer Viskosität von 15 000 mPas oder Methylhydroxypropylcellulose mit einer Viskosität von 50 000 mPas enthalten, sind in Abbildung 2 dargestellt.

### Beispiel 5

Wie in Beispiel 2 beschrieben, wurden Tabletten mit einem mittleren Gewicht von 350 mg und folgender Zusammensetzung pro Tablette hergestellt:

| | |
|---|---|
| Tramadolhydrochlorid | 200 mg |
| Methylhydroxypropylcellulose Type 2208, 50.000 mPas (Hersteller: Shin Etsu) | 50 mg |
| Mikrokristalline Cellulose PH 101 | 91 mg |
| Hochdisperses Siliciumdioxid | 5 mg |
| Magnesiumstearat | 4 mg |

Die in-vitro-Freisetzung des Wirkstoffes wurde gemäß Beispiel 1 untersucht, wobei folgende Freisetzungswerte (Mittelwerte aus n = 3) erhalten wurden:

| Zeit in Minuten | Freigesetzter Anteil in Gew.-% |
|---|---|
| 30 | 21 |
| 60 | 33 |
| 120 | 49 |
| 300 | 78 |
| 720 | 98 |

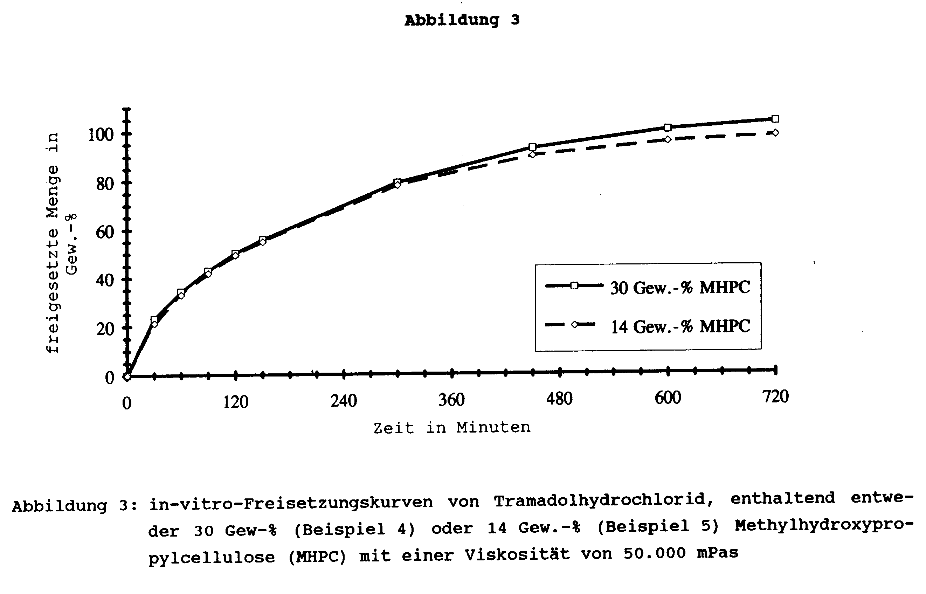

Die in-vitro-Freisetzungskurven der Tabletten, die entweder 50 mg, entsprechend 14 Gew.-%, oder 105 mg, entsprechend 30 Gew.-% (siehe Beispiel 4) Methylhydroxypropylmethylcellulose mit einer Viskosität von 50 000 mPas enthalten, sind in Abbildung 3 dargestellt.

### Beispiel 6

Matrixtabletten mit folgender Zusammensetzung pro Tablette:

| | |
|---|---|
| Tramadolhydrochlorid | 100 mg |
| Methylhydroxypropylcellulose Type 2910, 10.000 mPas (Hersteller: Dow Chemical Company) | 40 mg |
| Mikrokristalline Cellulose PH 101 | 26 mg |
| Hochdisperses Siliciumdioxid | 2 mg |
| Magnesiumstearat | 2 mg |

wurden in einer Ansatzgröße von 510 g gemäß Beispiel 2 hergestellt. Die erhaltenen Tabletten hatten einen Durchmesser von 8 mm, einen Wölbungsradius von 7,5 mm und ein mittleres Gewicht von 170 mg.

Die in-vitro-Freisetzung des Wirkstoffes wurde gemäß Beispiel 1 untersucht. Es wurden folgende Freisetzungswerte (Mittelwerte aus n = 2) erhalten:

| Zeit in Minuten | Freigesetzter Anteil in Gew.-% |
|---|---|
| 30 | 25 |
| 60 | 40 |
| 120 | 59 |
| 300 | 89 |
| 720 | 105 |

### Beispiel 7

Matrixtabletten mit folgender Zusammensetzung pro Tablette:

| | |
|---|---|
| Tramadolhydrochlorid | 150 mg |
| Hydroxypropylcellulose 30.000 mPas (Klucel® HXF) (Hercules, Düsseldorf) | 105 mg |
| Mikrokristalline Cellulose PH 101 | 86 mg |
| Hochdisperses Siliciumdioxid | 5 mg |
| Magnesiumstearat | 4 mg |

wurden gemäß Beispiel 2 in einer Ansatzgröße von 350 g hergestellt. Die in-vitro-Freisetzungsuntersuchung gemäß Beispiel 1 lieferte folgende Werte (Mittelwerte aus n = 2):

| Zeit in Minuten | Freigesetzter Anteil in Gew.-% |
|---|---|
| 30 | 25 |
| 60 | 35 |
| 120 | 50 |
| 300 | 75 |
| 720 | 100 |

### Beispiel 8

Matrixtabletten mit folgender Zusammensetzung pro Tablette:

| | |
|---|---|
| Tramadolhydrochlorid | 150 mg |
| Hydroxyethylcellulose 100.000 mPas (Natrosol® HHX) (Hercules, Düsseldof) | 105 mg |
| Mikrokristalline Cellulose PH 101 | 86 mg |
| Hochdisperses Siliciumdioxid | 5 mg |
| Magnesiumstearat | 4 mg |

wurden gemäß Beispiel 2 in einer Ansatzgröße von 350 g hergestellt. Die in-vitro-Freisetzungsuntersuchung gemäß Beispiel 1 lieferte folgende Werte (Mittelwerte aus n = 2):

| Zeit in Minuten | Freigesetzter Anteil in Gew.-% |
|---|---|
| 30 | 20 |
| 60 | 32 |
| 120 | 48 |
| 300 | 75 |
| 720 | 100 |

## Patentansprüche

1. Arzneimittel in Tablettenform mit verzögerter Wirkstofffreisetzung, enthaltend als Wirkstoff mindestens ein feuchtigkeitsunempfindliches, physiologisch verträgliches Salz von Tramadol und als pharmazeutisch akzeptablen Matrixbildner mindestens einen Celluloseether und/oder Celluloseester, der in einer 2 gew.-%igen wäßrigen Lösung bei 20° C eine Viskosität zwischen 3.000 und 150.000 mPas aufweist.

2. Arzneimittel nach Anspruch 1, dadurch gekennzeichnet, daß es als pharmazeutisch akzeptablen Matrixbildner mindestens einen Celluloseether und/oder Celluloseester mit einer Viskosität zwischen 10.000 und 150.000 mPas in einer 2 gew.-%igen wäßrigen Lösung bei 20° C enthält.

3. Arzneimittel nach einem oder beiden der Ansprüche 1 bis 2, dadurch gekennzeichnet, daß es als pharmazeutisch akzeptablen Matrixbildner mindestens eine Substanz ausgewählt aus der Gruppe der Methylhydroxypropylcellulosen, Hydroxyethylcellulosen, Hydroxypropylcellulosen, Methylcellulosen, Ethylcellulosen und Carboxymethylcellulosen enthält.

4. Arzneimittel nach einem oder mehreren der Ansprüche 1 bis 3, dadurch gekennzeichnet, daß es als pharmazeutisch akzeptablen Matrixbildner mindestens eine Substanz ausgewählt aus der Gruppe der Methylhydroxypropylcellulosen, Hydroxyethylcellulosen und Hydroxypropylcellulosen enthält.

5. Arzneimittel nach einem oder mehreren der Ansprüche 1 bis 4, dadurch gekennzeichnet, daß der verzögert freizusetzende Wirkstoffgehalt zwischen 10 und 85 Gew.-% und der Gehalt an pharmazeutisch akzeptablem Matrixbildner zwischen 10 und 40 Gew.-% liegt.

6. Arzneimittel nach einem oder mehreren der Ansprüche 1 bis 5, dadurch gekennzeichnet, daß der verzögert freizusetzende Wirkstoffgehalt zwischen 25 und 70 Gew.-% und der Gehalt an pharmazeutisch akzeptablem Matrixbildner zwischen 10 und 40 Gew.-% liegt.

## Claims

1. A drug in tablet form which exhibits delayed release of an active ingredient, and which contains at least one moisture-insensitive, physiologically compatible salt of tramadol as an active ingredient and at least one cellulose ether and/or cellulose ester, which has a viscosity between 3000 and 150,000 mPas in a 2 % by weight aqueous solution at 20°C, as a pharmaceutically acceptable matrix former.

2. A drug according to claim 1, characterised in that it contains at least one cellulose ether and/or cellulose ester which has a viscosity between 10,000 and 150,000 mPas in a 2 % by weight aqueous solution at 20°C as a pharmaceutically acceptable matrix former.

3. A drug according to one or both of claims 1 to 2, characterised in that it contains at least one substance selected from the group comprising methylhydroxypropyl celluloses, hydroxyethyl celluloses, hydroxypropyl celluloses, methyl celluloses, ethyl celluloses and carboxymethyl celluloses as a pharmaceutically acceptable matrix former.

4. A drug according to one or more of claims 1 to 3, characterised in that it contains at least one substance selected from the group comprising methylhydroxypropyl celluloses, hydroxyethyl celluloses and hydroxypropyl celluloses as a pharmaceutically acceptable matrix former.

5. A drug according to one or more of claims 1 to 4, characterised in that the content of delayed-release active ingredient is between 10 and 85 % by weight and the content of pharmaceutically acceptable matrix former is between 10 and 40 % by weight.

6. A drug according to one or more of claims 1 to 5, characterised in that the content of delayed-release active ingredient is between 25 and 70 % by weight and the content of pharmaceutically acceptable matrix former is between 10 and 40 % by weight.

## Revendications

1. Médicament sous forme de comprimé à libération retardée de substance active, contenant comme substance active au moins un sel de tramadol acceptable du point de vue physiologique, insensible à l'humidité, et comme matière formant une matrice acceptable du point de vue pharmaceutique, au moins un éther de cellulose et/ou un ester de cellulose, qui présente en solution aqueuse à 2 % en poids une viscosité à 20 °C comprise entre 3 000 et 150 000 mPas.

2. Médicament suivant la revendication 1, caractérisé en ce qu'il contient comme matière formant une matrice acceptable du point de vue pharmaceutique au moins un éther de cellulose et/ou un ester de cellulose ayant une viscosité comprise entre 10 000 et 150 000 mPas, mesurée à 20 °C sur une solution aqueuse à 2 % en poids.

3. Médicament suivant l'une des revendications 1 et 2 ou les deux, caractérisé en ce qu'il contient comme matière formant une matrice acceptable du point de vue pharmaceutique au moins une substance choisie dans le groupe des méthylhydroxypropylcelluloses, des hydroxyéthylcelluloses, des hydroxypropylcelluloses, des méthylcelluloses, des éthylcelluloses et des carboxyméthylcelluloses.

4. Médicament suivant une ou plusieurs des revendications 1 à 3, caractérisé en ce qu'il contient comme matière formant une matrice acceptable du point de vue pharmaceutique au moins une substance choisie dans le groupe des méthylhydroxypropylcelluloses, des hydroxyéthylcelluloses et des hydroxypropylcelluloses.

5. Médicament suivant une ou plusieurs des revendications 1 à 4, caractérisé en ce que la teneur en substance active à libération retardée se situe entre 10 et 85 % en poids et la teneur en matière formant une matrice acceptable du point de vue pharmaceutique se situe entre 10 et 40 % en poids.

6. Médicament suivant une ou plusieurs des revendications 1 à 5, caractérisé en ce que la teneur en matière à libération retardée est comprise entre 25 et 70 % en poids et la teneur en matière formant une matrice acceptable du point de vue pharmaceutique est comprise entre 10 et 40 % en poids.
